# EUROPEAN PATENT APPLICATION

(11) **EP 2 055 350 A2**
(43) Date of publication of application: **06.05.2009**
(21) Application number: 08253543.6
(22) Date of filing: 30.10.2008
(51) Int. Cl.: A61Q 9/00, A61K 8/92

(54) **Epilatory product comprising an air-activated heat-generating module**

(30) Priority: 31.10.2007 US 930641
(71) Applicant: MCNEIL-PPC, INC., Skillman, NJ 08558 (US)
(72) Inventor: Luizzi, Joseph M., Newtown, PA 18940 (US)
(74) Representative: Kirsch, Susan Edith

(57) **Abstract**

Embodiments of the invention relate to a packaged epilation product comprising an epilation composition formed on a substrate and an air-activated heat-generating module.

## Description

### FIELD OF THE INVENTION

The present invention relates to methods, compositions and products useful for epilation and, in particular, to epilation products including an epilation composition and a heat-generating module.

### BACKGROUND OF THE INVENTION

For aesthetic or personal reasons, many people find it desirable to remove unwanted hair from various areas of the human body, e.g., the bikini area, or areas of the face such as around the eyebrows or lips. Methods known for removing hair include shaving, chemical treatment, and physical hair removal. Shaving the hair suffers from the inconvenience of frequently needing to repeat the process, e.g., daily. The use of chemical treatments to cleave the hair shaft (i.e., depilatories) suffers from the drawback of using the chemicals themselves, which generally irritate the skin, have unpleasant odors, and are messy.

Physical hair removal, in which hair is pulled from the skin "by the roots" (broadly termed "epilation") generally overcomes the problems of chemical irritation, smell, and the requirement for frequent treatment, but additional problems are introduced. The epilation of hair using "waxes" and sugars has been known in literature for years. In order to remove the unwanted hair, the epilation composition is heated and applied to the skin, and while still in its molten state, a fabric backing is applied to the molten epilation composition. Once cooled, the fabric backing is pulled, removing the epilation composition and the hair encapsulated within, thus denuding the skin surface.

To address the poor hair removal problems of ready to use products, products that include an epilation composition that requires heating (a so-called "hot wax") prior to use have been proposed. However, to render a conventional hot wax fluid enough to spread across the skin, the hot wax needs to be heated to a high temperature. This heating step generally requires the use of a microwave, often inconveniently located away from where the individual desires to perform the epilation process. Furthermore, the results of the heating are often unpredictable, usually resulting in not enough heating, thereby causing the individual to either use a wax that cannot spread properly or forcing the user to heat the wax again. Another possible unfortunate result is too much heating of the hot wax, resulting in burning of the skin or forcing the user to wait to let the wax cool down.

In order to provide consumer convenience, ready-to-use products (also known as "cold waxes") are available. While these products include an epilation composition that does not require heating, cold waxes have other drawbacks. They are typically quite tacky in nature, and as they require good flow characteristics at room temperature, so as to encapsulate the hair, they often lack sufficient modulus. Consequently, they often fail cohesively, leaving waxing compound residue on the skin, or worse yet, failing to remove the hair.

Manufacturers have added additional convenience to cold waxes by having the compound pre-applied to the backing substrate. However, cold waxes suffer from so-called "cold flow" problems, i.e., the epilation composition is fluid enough to flow in the package, prior to use. This often results in sticky, messy, or even unusable product.

As such, Applicants have identified the need for an epilation product that includes both convenient, pre-coated epilation strips as well as means to heat the strips. Furthermore, Applicants have surprisingly discovered that this need may be met by an epilation product that includes an air-activated, heat-generating module and an epilation composition formed on a substrate.

### SUMMARY OF THE INVENTION

According to a first aspect of the present invention, there is provided a packaged epilation product comprising an epilation composition formed on a substrate, and an air-activated heat-generating module.

According to a second aspect of the present invention, there is provided a method of removing hair. The method includes separating an air-impermeable barrier wrap from an air-activated, heat generating component to expose said air-activated, heat generating component to enhanced air flow, placing the exposed air-activated, heat generating component into heat transfer proximity with an epilation composition formed on a substrate for a time sufficient to render the epilation composition tacky, and contacting the hair with said tacky epilation composition in a manner sufficient to remove said hair.

### BRIEF DESCRIPTION OF THE DRAWINGS

Examples of embodiments of the present invention will now be described with reference to the drawings, in which:
FIG. 1 depicts a top plan view of various components of an epilatory product in accordance with embodiments of the invention;
FIG. 2 depicts a schematic, cross-sectional view of an epilatory strip in accordance with embodiments of the invention;
FIG. 3 depicts a schematic, cross-sectional view of an air-activated heat-generating module in accordance with embodiments of the invention;
FIG. 4 depicts a schematic cross-sectional view of an alternative embodiment of the air-activated heat-generating module in accordance with embodiments of the invention;
FIG. 5 depicts an insulating pouch that may be included with the epilatory product in accordance with embodiments of the invention; and
FIG. 6 depicts the insulating pouch of **FIG. 5** having an epilatory strip placed therein, in accordance with embodiments of the invention.

### DETAILED DESCRIPTION OF THE INVENTION

The present invention provides products, methods, and materials useful for epilation. In particular, the compositions, methods, and materials of the present invention provide the combination of excellent hair removal as well as an ease of use that is lacking in conventional epilation systems.

By "epilation" it is meant the physical and typically forceful removal of the entire hair shaft hair from the body. Epilation includes what is commonly referred to as "waxing", that is, applying an epilation composition that bonds to the hair, then applying sufficient force to extract the hair from the body. Epilation compositions may include, for example waxes, polymers, resins, rosin or other bonding materials, optionally compounded with oils, fillers, dyes, and the like. Epilation compositions may be heated, even melted, to provide sufficient tack for hair removal.

According to embodiments of the present invention, a body surface undergoes epilation. By "body surface" it is meant that portion of the body encompassing a surface of the body from which unwanted hairs protrude (i.e., skin) and/or the hairs protruding therefrom. Examples of body surfaces include the bikini area, legs, arms and/or areas of the face such as around the eyebrows or lips.

Applicants have recognized that conventional epilation products fail to provide the combination of (1) the convenience of pre-coated strips that are not prone to melting prematurely and thereby forming a mess and/or ruining the product; (2) have a portable, easy-to-use heating source that is not prone to leaking onto or wetting the other components of the epilation product; and (3) have excellent hair removal. In response to Applicants recognition of this problem, Applicants provide in the present invention, an epilation product that includes an epilation composition formed on a substrate and an air-activated, heat-generating module.

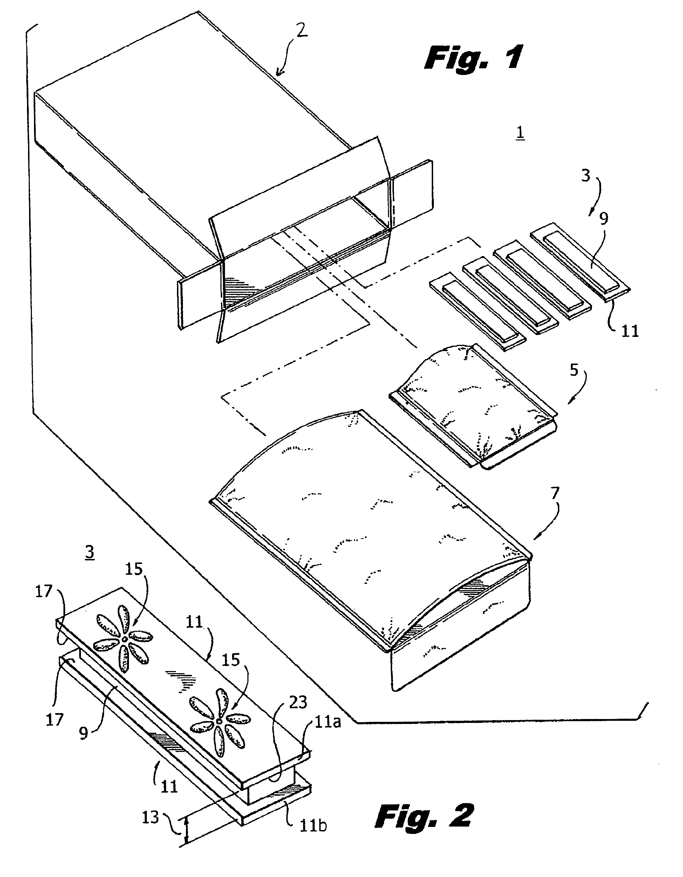

Referring to FIGS. 1-5, there is shown a first embodiment of the present invention, an epilation product 1. FIG. 1 depicts a top plan view of various components of the epilation product 1 in accordance with embodiments of the invention. The components of the epilation product 1 may be packaged together, such as in a box or carton 2, together with instructions regarding how to use the product to remove unwanted hair. The epilation product 1 includes one or more epilation strips 3, an air-activated heat-generating module 5, and an optional thermally insulating pouch 7.

FIG. 2 depicts a cross-sectional view of a suitable configuration for epilation strip 3. Epilation strip 3 includes an epilation composition 9 formed on a substrate 11. The epilation composition may be formulated such that at relatively low temperatures the epilation composition resists flow and may be relatively low in tack. By "relatively low temperatures" it is meant, temperatures from about ambient temperature, 20°C, to temperatures that may be experienced during storage and shipping of the product, e.g., as high as about 35°C to perhaps, in certain embodiments, as high as about 45°C. Furthermore, the epilation product is generally designed such that at elevated temperature, the epilation composition becomes more fluid and more tacky than at relatively lower temperatures. By "elevated temperature," it is meant a temperature above the relatively low temperature described above, yet one that is still suitable for contact with the body, e.g., from about 35°C to about 50°C. By way of another example, the epilation composition may be designed to be resistant to flow and have relatively high cohesive strength at a relatively low temperature in the range of 30-35°C, and exhibit increased tack and fluidity as well as reduced cohesive strength at an elevated temperature that is in the range of 35-45°C.

The epilation composition may include any of various components used in conventional epilation compositions, e.g., those sometimes referred to as "waxes" or, in particular, those sometimes referred to as "hot waxes," those epilation products designed to be heated prior to use.

The epilation composition generally comprises a tackifying material to help the epilation composition adhere to the hair to be epilated, as well as to perhaps provide some melt stiffness and resistance to fluidity at elevated temperature. Desirably the tackifying material is a rosin or sugar-based material, although a wide range of tackifying materials, examples of which are discussed below are contemplated.

Suitable tackifying materials include, for example, hydrophilic materials such as sugar-based materials (e.g., polysaccharides such as sucrose, fructose, honey, and the like) or mixtures of a water soluble polymer such as polyvinyl alcohol and a gel-promoting agent such as boric acid.

Other suitable tackifying materials include polymeric or oligomeric materials such as tackifying aliphatic or aromatic resins or tackifying block polymers. Suitable tackifying resins include rosins (i.e., rosinaceous materials), for example rosin or a rosin ester. The tackifying material is desirably phase compatible with other ingredients in the epilation composition and the tackifying material may have a melting point (in its pure state) greater than about 55°C, such as from about 65°C to about 100°C. The tackifying resins may have a weight average molecular weight that is greater than about 300, such as from about 400 to about 1000.

Examples of suitable tackifying resins include hydrophobic tackifying resins such as hydrocarbon resins such as rosins, glyceryl rosinate and other terpene resins; cyclic aliphatic resins; other tackifying resins known in the art, as well as certain high melting point "microcrystalline waxes".

The concentration of tackifying material may be high enough to provide adhesion to the hair, particularly when heated. In one embodiment the concentration of tackifying resin in the epilation composition is from about 10% to about 70%, such as from about 30% to about 60% (note that all percentages listed herein are percentages by weight unless otherwise stated).

The epilation may further include any number of polymers suitable for providing cohesive strength, such as those polymers typically used to provide cohesive strength in adhesive compositions. Suitable examples include acrylics; block copolymers such as those based on styrene and isoprene or butadiene and polyolefins (e.g., EVA, LDPE, APP, EMA, etc.).

The epilation composition may include other components such as diluents. In one embodiment the epilation composition includes a diluent to aid in delivering the composition across an expanse of skin that is to be epilated. The diluent is generally selected to be non-irritating to the skin and readily dissolves, disperses or suspends other ingredients in the composition. In preferred embodiments of the invention, the epilation composition has a high concentration or predominance of hydrophobic ingredients. In this embodiment, the diluent is preferably selected so as to be hydrophobic. Conversely, for compositions in which there is a high concentration or predominance of hydrophilic ingredients, the diluent may be selected so as to be hydrophilic. The diluent may be volatile, moderately volatile, or non-volatile at room temperature. The diluent may be selected from a liquid diluent, a solid diluent, or combinations thereof.

The liquid diluent is a liquid at room temperature. In one embodiment of the invention the liquid diluent is a hydrophobic liquid. Suitable examples include mineral oils; silicone oils; vegetable oils; or other volatile or non-volatile hydrophobic liquids such as liquid turpenes and the like. Other liquid hydrophobic diluents that have some polarity may also be suitable. Suitable examples of such include rosin esters such as triethyleneglycol rosinate, esters of fatty acids (e.g., triglycerides), aldehydes, ketones, phenols. In another embodiment the liquid diluent is a hydrophilic liquid at room temperature such as water, propylene glycol, water-miscible alcohols. In one embodiment, the epilation composition is substantially free (i.e., having less than about 5%, preferably less than about 1 %) of hydrophilic liquids such as water.

The liquid diluent may be present in a concentration that does not interfere with the ability of the composition to provide tack in use. In one embodiment of the invention, the liquid diluent is hydrophobic and is may be present in a concentration that is from about 0% to about 80%

The solid diluent is a solid, paste, gel, or the like at room temperature and is generally a non-polymerized or moderately polymerized material. Particularly notable solid diluents are hydrophobic solid diluents such as fats, i.e., glyceryl esters of fatty acids and waxes, i.e., mixtures of esters, not necessarily esters of glycerol, often including long chain alkyl groups; or mineral waxes such as paraffin wax or petrolatum; and silicone waxes (derived from silicon, oxygen, oxygen, and hydrogen) such as alkylmethyl-dimethylsiloxanes. The solid diluent may melt over a temperature range that is within from about 30°C to about 75°C, preferably from about 35°C to about 65°C. Suitable solid diluents include, for example, petrolatum. Note that while hydrophobic solid diluents are particularly notable, a hydrophilic solid diluent such as hydrophilic film-forming polymer (e.g., polysaccharides such as starches, cellulose polymers, gums, and the like) is also contemplated, particularly for embodiments in which the epilation composition is aqueous, i.e., has water as a continuous phase.

The solid diluent may be present in a concentration that does not interfere with the ability of the composition to provide tack in use. In one embodiment of the invention, the concentration of solid diluent, preferably a hydrophobic solid diluent, more preferably a wax, is from about 0% to about 50%, preferably from about 5% to about 35%.

The epilation composition may also include ingredients that provide beneficial biological/biochemical activity to the subject, such as those that may be suitable for retarding hair growth (e.g., extracts of soy for example as disclosed in U.S. Patent No. 6,55,143) chemically cleaving hair shafts (e.g., thioglycolates), anti-inflammatory agents e.g., benoxaprofen, centella asiatica, bisabolol, extracts of feverfew, green tea extract, green tea concentrate, hydrogen peroxide, salicylates, oat oil, chamomile, dipotassium glycyrrhizate), anti-edema agents (e.g., bisabolol natural, synthetic bisabolol, corticosteroids, beta-glucans), analgesics (e.g., for example, "caine" molecules such as benzocaine, dibucaine, lidocaine; benzyl alcohol, camphor, juniper tar, menthol, methyl nicotinate, methyl salicylate, phenol, resorcinol), counterirritants (e.g., allantoin, camphor, menthol, methyl salicylate, peppermint and clove oils, ichtammol).

The epilation composition may also include particulate materials dispersed therein to provide desirable texture, rheology, or reduced material cost. Suitable particulate materials include inorganic particulates such as various refined minerals, oxides, silicates, and the like. Examples include silicon oxides, aluminum oxides, zinc oxides, titanium oxides, boron nitrides, talc, gypsum, calcite among others, and combinations thereof. Other suitable particulate materials include organic particulates such as synthetic polymers that may be chemically crosslinked such as silicone elastomers, natural particulate materials including proteins or polysaccharides derived from vegetable or animal sources. The particulate material may be coated such as with silanol or hydrophobic moieties in order to enhance compatibility with the remainder of the epilation formulations. The particulate material may be present in any suitable concentration, such as from about 0% to about 10%.

The epilation composition may include other functional components such as dyes, fragrances, essential oils, pH adjusters (for aqueous compositions), dispersants, emulsifiers, wetting agents, rheology modifiers, preservatives, sequestering agents, and antioxidants.

To also provide ease of heating, the epilation composition may be formed to a thickness that renders it suitable for relatively fast heating. As such, as shown in FIG. 2, the epilation composition may be formed to a thickness 13 that is, for example, less than about 5 milimeters (mm), such as less than about 3mm. The thickness 13 may be significantly smaller than other dimensions (e.g., a length and/or width) of the epilation composition. For example, the thickness 13 of the epilation composition may be less than about 10% of the length and/or width of the epilation composition, such as less than about 5% of said length or width. The epilation composition may have an area density (basis weight) that is about 50 grams per square meter (gsm) to about 500 gsm, more preferably from about 100 gsm to about 300 gsm.

In order to provide ease of delivering the epilation composition to the body surface that requires epilation, and also to also provide ease of heating the epilation compostion, the epilation composition is formed on a substrate 11. The substrate may have one or more desirable features, including: water-resistance or water-insolubility to prevent the substrate from degrading prior to use; small size, light weight or minimal thickness for convenience; flexibility such that the substrate is readily flexed across curved areas of the body; wettability with the epilation composition for ease of coating; thermal resistance to prevent thermal degradation of the substrate in use.

The substrate 11 may include fibrous materials such as paper, non- woven, or woven fibers; thermoplastic films, and combinations or laminates thereof. The substrate 11 may include a surface that has been rendered hydrophobic or hydrophobic as needed to render the substrate 11 wettable to the epilation composition. Suitably the substrate 11 is a flexible sheet material capable of carrying the layer of epilation composition 9. The sheet material may be porous, absorbent and/or fibrous such that the epilation composition is partially absorbed into the carrier to provide anchorage of the layer on the substrate. Suitably the epilation composition 9 and substrate 11 are firmly attached such that in use, the epilation composition 9 and substrate 11 do not separate from one another.

In one embodiment, the substrate 11 includes nonwoven fibers such as those derived from cellulosic material such as wood pulp, cotton, hemp, jute, flax and mixtures thereof. In another embodiment, the substrate 11 includes fibers or films derived from rayon, polyesters, polyurethanes, polyvinyl acetate, polyacrylates, polymethacrylates, polyamides, styrenes, styrene copolymers, polyolefins, polyvinylchlorides, inorganic fibres, polyethylene, polypropylene, polyethylene terephthalate, nylons, cellophane, and mixtures and copolymers thereof.

The substrate 11 may comprise more than one layer of sheet material. Each layer of sheet material may be constructed from the same or different materials. Suitably each layer is laminated to an adjacent layer.

Referring again to FIG. 1, while the shape of the substrate 11 is depicted as rectangular, this is not required. The shape of a substrate 11 may correspond to the shape of the portion of the body from which the hair is to be removed, e.g., the substrate 11 may include curved portions, such as may be suitable for removing hair from above the upper lip, near the eyebrows, or the bikini area. The epilation product may include substrates 11 of varying shapes, such that the user can select the appropriate shape for the particular body part in need.

As shown in FIG. 2, the epilation composition 9 may be formed between two individual substrate layers, e.g., a top substrate layer 11 a and a bottom substrate layer 11b. In this particular configuration, the individual substrate layers 11a, 11b serve to prevent hot epilation composition from inadvertantly sticking to a surface prior to be used on the body. After heating, the user pulls the individual substrate layers 11a, 11b apart, yielding two distinct epilation composition/substrate pairs that can each be used for epilation. Various alternative configurations of the substrate 11 and the epilation composition 9 are contemplated. For example, in one embodiment, top substrate layer 11a is absent. In another embodiment the substrate 11 is folded.

The epilation composition 9 may be formed on all or only selected regions of the substrate 11. To facilitate grasping the substrate without getting one's hands in contact with tacky epilation composition, edge portions 17 of the substrate may be free of epilation composition 9. The remainder of the substrate may have epilation composition 9 formed thereon. In another embodiment, the epilation composition 9 may be formed on the substrate in a pattern, e.g. finely-spaced dots, stripes, or another pattern. The epilation composition 9 may be formed on the substrate 11 by coating techniques such as slot coating, roll coating, spraying, and the like.

In another embodiment of the invention, the substrate includes a temperature-indicating material designed to provide a sensation that varies with temperature. For example, the temperature-indicating material may be a thermochromic material, i.e., a material that changes color or visual appearance upon being heated. It is further desirable that the thermochromic material returns to its original appearance upon being cooled, i.e., the visual appearance change is reversible.

In one notable embodiment, the thermochromic material is designed to undergo a discernible color change when subject to a temperature transition that occurs between about 35°C and about 50°C, such as between about 40°C and about 50°C.

In one embodiment, the temperature-indicating material is formed as one or more thermochromic layers 15 on the substrate 11. The thermochromic layer may include a thermochromic material as well as any suitable binders or resins that provide features such as compatibility with the thermochromic material, good adhesion to the substrate 11 and good image formation. Suitable thermochromic materials include inks based on thermochromic chemistries, such as those utilizing cholesteric, ester or acrylic liquid crystal phase change materials. Commercial examples of these include ThermaSOLV W/B Non-woven Inks from AIC/Sun Chemical (Saint Aignan de Grand Lieu, France) and Waterbased Flexo Inks from Chromatic Technologies, Inc. (Colorado Springs, CO).

In order to facilitate viewing of the thermochromic layer 15, the thermochromic layer 15 may be applied on a surface 21 of the substrate 11 that is opposite to a first surface 23 on which the epilation composition is formed. The thermochromic layer 15 may define one or more discrete images, e.g. flowers, butterflies, or other such images. The thermochromic layer 15 may be applied to the substrate via any suitable means (e.g., flexographic, gravure, or ink jet printing).

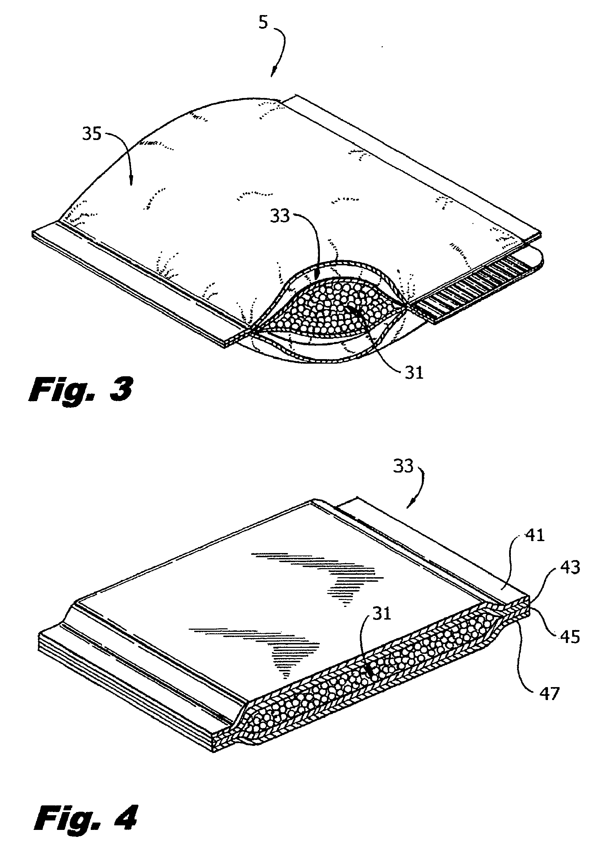

The epilation product 1 further includes an air-activated, heat-generating module. A suitable configuration of the air-activated, heat-generating module is depicted in FIG. 3. Air-activated, heat-generating module 5 includes an air-activated, heat-generating component 31, enclosed in an air-impermeable, barrier wrap, 35.

The air-activated, heat-generating component 31 releases heat upon exposure to air, such as, via an exothermic reaction with oxygen or nitrogen. In one embodiment, the air-activated, heat-generating component 31 is a finely divided solid such as a powder or granular material. Suitable materials include oxidizable metals such as iron. In order to enhance or speed-up the exothermic reaction, the air-activated, heat-generating component 31 may also include a water-retaining material such as a powdered or granular mineral, e.g., vermiculite, charcoal or diatmoaceous earth or wood powder, that has some residual water absorbed thereon. The air-activated, heat-generating component 31 may further include a catalyst suitable for catalyzing the exothermic reaction, such as activated carbon and/or inorganic salts such as sodium chloride, potassium chloride, or magnesium chloride.

The amount of air-activated, heat-generating component 31 may be selected in order to provide a sufficient amount of heat in order to raise the temperature of epilation composition 9 from room temperature to above 45°C in about 3-15 minutes. For example, when using a mixture of iron powder and activated carbon, one may use about 20 grams to about 40 grams, for example about 30 grams of air-activated, heat-generating component. The ratio of iron to activated carbon to granular material to water is selected in a manner known in the art of portable chemical heat generating technology. Suitable materials and methods are disclosed in U.S Patent 6,453,648 to Zars, Inc.; and U.S Patent 5,046,479 to Mycoal Body Warmers Co., both of which are herein incorporated by reference.

The air-activated, heat-generating component 31 may be isolated from air prior to use, such as by enclosing it in air-impermeable, barrier wrap, 35. Air-impermeable, barrier wrap, 35 may be formed from any suitable material that is impermeable to air. The air-impermeable, barrier wrap, 35 is desirably strong enough resist inadvertent tearing and also light weight and relatively thin. Particularly suitable materials include plastics such as polyethylene or polypropylene.

In order to contain the air-activated, heat-generating component 31 once the air-impermeable, barrier wrap, 35 is removed, the air-activated, heat-generating component 31 may be contained in an air-permeable container 33. The air-permeable container 33 is designed to provide one or more of the following functions: (1) provide some space between the air-activated, heat-generating component 31 and the epilation composition 9 to be heated; (2) transfer air from the outside environment into contact with the air-activated, heat-generating component 31; (3) transfer heat from the air-activated, heat-generating component 31 to the surrounding air; and (4) for embodiments in which the air-activated, heat-generating component 31 is a finely divided solid, contain the air-activated, heat-generating component 31. In one embodiment, the air-permeable container 33 may have an air-permeability in a flattened state that is from about 500 to 50000 seconds per hundred cubic centimeters (sec/100cc), more preferably 5000 to 10000 sec/100 cc.

As such, the air-permeable container 33 desirably includes one more material layers, such as (1) a microporous film, such as a microporous or fmely perforated thermoplastic film, e.g., polyethylene; (2) a fibrous layer, such as a layer of nonwoven fibers (polypropylene, rayon, or similar materials) oriented by, for example, carding, and bonded via heat, chemical or other means; or a laminate of (1) and (2). The air-permeable container 33 may also include a pigmented plastic film, such as a white, pigmented polyurethane or polypropylene in order to mute or hide the appearance of the air-activated, heat-generating component 31.

FIG. 4 depicts an embodiment of air-permeable container 33 in which the air-impermeable barrier wrap 35 has been removed. In this particular embodiment, air-permeable container 33 is multilayered and includes a top nonwoven layer 41, a top microporous film layer 43, a bottom pigmented film 45, and a bottom nonwoven layer 47. The air-activated, heat-generating component 31 is positioned between top microporous film layer 43 and bottom pigmented polyurethane film 45. Note that in certain "asymmetrical" embodiments, bottom pigmented film 45 need not be microporous, in which case the air-flow and heat-flow will be preferentially through top layers 41, 43.

Preferably the length and width dimensions of the heat-generating module, as viewed from the top may range may be from about 5cm to about 10 cm, more preferably from about 7 cm to about 15cm. One particularly suitable air-activated, heat-generating module is commercially available from Okamoto Industries of Stratford, CT as BODI-HEAT warmers.

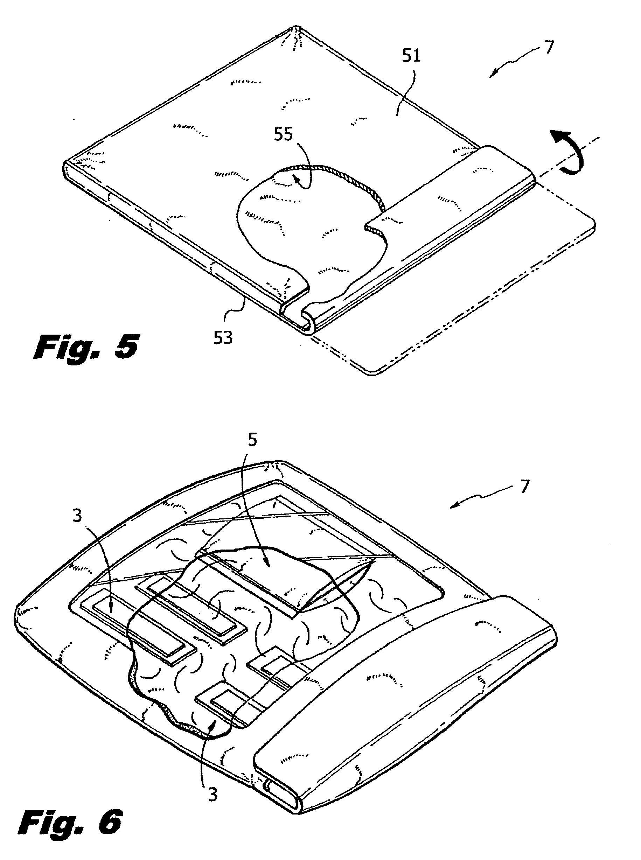

FIG. 5 depicts a schematic, cross-sectional view of insulating pouch 7, which may be used in accordance embodiments of the invention. Insulating pouch 7 functions to trap the heat that is generated by the heat-generating module 5 such that it can be efficiently used to heat the epilation composition 5. Insulating pouch 7 may be formed from flexible, compliant materials, and/or light-weight materials that will not be damaged from heat generated by the heat-generating module 5. The insulating pouch 7 may include fibrous materials, such those including or formed from non-woven fibers such as fibers of cellulose, olefin, polyester rayon, and the like. The nonwoven fibrous material may have a basis weight that is from about 5gsm to about 200gsm, preferably from about 10 gsm to about 150 gsm, more preferably from about 20 gsm to about 100gsm. Other material layers, such as metal foil, cardboard, thermoplastic film, and combinations thereof may be included as well.

Insulating pouch 7 is sized such that it can folded or (either loosely or tightly) sealed around one or more epilation strips 3. In one embodiment, as shown in FIG. 5, insulating pouch 7 is a simple construction that has a pocket 55 that is defined by a top flap 51 and a bottom flap 53. The top flap 51 is such that gravity causes top flap to droop down over the pocket 55, thereby providing thermal insulation. Other constructions for insulating pouch 7 are however contemplated, including those with loop engageable seals (e.g., VELCRO) or zippers; or those such as foil-lined cardboard trays that have one or more openings for placing a heat-generating module and epilation strips and inside a chamber to contain heat.

In certain embodiments, the insulating pouch 7 is such that a change in color of thermochromic layer 15 present on epilatory strip 3 can be seen through the insulating pouch 7. This facilitates the user knowing when the epilation strips are ready to use. In this particular embodiment, the insulating pouch consists essentially of transparent or semitransparent materials such as thermoplastic films or non-woven fibrous materials, or combinations thereof, such as those free of pigments.

In order to use the epilation product according to embodiments of the invention defined herein. A user removes the components from a package or carton. The user separates any air-impermeable barrier wrap to expose the air-activated heat-generating module to enhanced air flow. This exposed air-activated, heat-generating module is then placed in close proximity to one or more epilation strips (i.e., a substrate that is coated with the epilation composition) for a period of time sufficient to render the epilation composition more fluid and/or tacky. For example, the epilation strip may be placed in thermally insulating pouch. In embodiments wherein the substrate has thermochromic material applied on it, the heat may be applied for a time period sufficient to effect a change in visual appearance or color of the thermochromic material (signaling to the user enough heat has been applied).

The epilation strip is then flexed around curved areas of the skin and contact is provided between the hairs to be removed and the epilation composition. To enhance contact between the epilation composition and the hairs, the substrate may be pressed firmly on to the skin for a period from a few seconds to as long as about one minute. For embodiments wherein the epilation composition includes the thermochromic material, the user may wait a period of time for an appearance change in the thermochromic material (indicating sufficient cooling has taken place to promote good hair removal). The substrate is then removed in a manner sufficient to epilate the skin, leaving hairs attached to the coating of epilation composition.

The advantages of the invention and specific embodiments of the skin care compositions prepared in accordance with the present invention are illustrated by the following examples.

### Example 1

An epilation strip, CAREFREE Face & Bikini Total Wax Strip KIT, similar to the epilation strip described with reference to FIG. 2, commercially available from McNeil-PPC of New Brunswick, NJ was placed inside a thermally insulating pouch constructed of a non-woven fibrous material and having a shape and configuration similar to that shown in FIG. 5. An air-activated heat generating module, commercially available from Okamoto Industries of Stratford, CT as BODI-HEAT warmer, similar to the air-activated heat generating module shown in FIG. 4, was separated from its air-impermeable, barrier wrap and also placed in the thermally insulating pouch for 15 minutes The epilation composition became more fluid and tacky. The substrates were peeled apart and the heated epilation compostion was placed in contact with a body surface having hair to be removed. The substrate and epilation composition were forcibly removed, having hair embedded therein.

## Claims

1. An epilation product, comprising:
an epilation composition formed on a substrate; and
an air-activated heat-generating module.

2. The epilation product of claim 1, wherein said heat-generating module comprises an air-activated, heat-generating component enclosed in an air-impermeable, barrier wrap.

3. The epilation product of claim 1, wherein said heat-generating module comprises an air-activated, heat-generating component enclosed in a air-permeable container, and wherein said air-permeable container is enclosed in an air-impermeable, barrier wrap.

4. The epilation product of any preceding claim, wherein said epilation composition comprises a rosin or sugar-based material.

5. The epilation product of any preceding claim, wherein said epilation composition has a thickness less than about 5 mm.

6. The epilation product of any preceding claim, wherein said air-activated, heat-generating component comprises a component that is capable of undergoing an exothermic reaction when exposed to air.

7. The epilation product of any preceding claim, wherein said air-activated, heat-generating component is a finely-divided solid.

8. The epilation product of any preceding claim, wherein said air-activated, heat-generating component comprises an oxidizable metal.

9. The epilation product of any preceding claim, wherein said epilation composition is formed between two substrates.

10. The epilation product of any one of claims 1 to 8, wherein said substrate has a thermochromic material formed thereon.

11. The epilation product of any one of claims 1 to 8, wherein said epilation composition is formed on a first surface of said substrate, and said substrate has a thermochromic material formed on second surface opposite to said first surface of said substrate.

12. The epilation product of any preceding claim, further comprising a thermally insulating pouch.

13. The epilation product of claim 12, wherein said thermally insulating pouch is sized to accommodate simultaneously said epilation composition formed on a substrate and said heat-generating module.

14. The epilation product of claim 12 or claim 13, wherein said thermally insulating pouch comprises a nonwoven material.

15. A method of removing hair, comprising:
separating an air-impermeable barrier wrap from an air-activated, heat generating component to expose said air-activated, heat generating component to enhanced air-flow;
placing said exposed air-activated, heat generating component into heat transfer proximity with an epilation composition formed on a substrate for a time sufficient to render the epilation composition tacky; and
contacting the hair with said tacky epilation composition in a manner sufficient to remove said hair.

16. The method of claim 15, wherein said placing of said exposed air-activated, heat generating component comprises placing an air-permeable container that contains said air-activated, heat generating component.

17. The method of claim 15, wherein said placing of said exposed air-activated, heat generating component and an epilation composition formed on a substrate into heat transfer proximity comprises placing said exposed air-activated, heat generating component and an epilation composition formed on a substrate into an insulating pouch.

18. The method of any one of claims 15 to 17, wherein said epilation composition comprises a rosin or a sugar-based material.

19. The method of any one of claims 15 to 18, wherein said epilation composition has a thickness less than about 5 mm.

20. The method of any one of claims 15 to 19, wherein said air-activated, heat-generating component undergoes an exothermic reaction when exposed to air after separating said air-impermeable barrier wrap from said air-activated, heat generating component.
